# EUROPEAN PATENT APPLICATION

(11) **EP 4 840 762 A1**
(43) Date of publication of application: **18.11.2026**
(21) Application number: 26176118.3
(22) Date of filing: 01.05.2026
(51) Int. Cl.: B64D 37/00, B64D 37/02, B64D 37/30, B64D 37/34, F02C 9/40, F02D 19/06, F02D 41/00

(54) **AIRCRAFT FUEL DOSING SYSTEM**

(30) Priority: 15.05.2025 GB 202507496
(71) Applicant: Airbus Operations Ltd, Filton, Bristol BS34 7PA (GB)
(72) Inventor: Edwards, Henry, Bristol, BS34 7PA (GB)
(74) Representative: Worthington, Richard Easton

(57) **Abstract**

The present invention relates to an aircraft fuel dosing system featuring a dosing control unit (DCU) configured to receive an output signal from at least one sensor, said signal being indicative of at least one parameter associated with the aircraft, and being further configured to automatically adjust a composition of a fuel mixture delivered to a fuel consuming device based on the received signal.

## Description

### FIELD OF THE INVENTION

The present invention relates to an aircraft fuel dosing system, to an aircraft comprising said aircraft fuel dosing system, and to a method of automatically adjusting a composition of a fuel mixture on-board an aircraft.

### BACKGROUND OF THE INVENTION

Aircraft fuel can be "dosed" with fuel additives prior to flight for several different reasons, such as enhancing fuel performance and/or improving the efficiency of the aircraft. The volume of fuel additive that is added to the fuel may also change depending on several factors, such as the flight route of the aircraft (to account for environmental changes and parameters) and the age of the aircraft (for maintenance purposes).

Fuel additives may be pre-mixed into the fuel from a refinery before being introduced into the aircraft. While this ensures a consistent and controlled fuel composition, it does not allow for customisation or adjustment of the fuel composition during the aircraft's flight.

It is therefore an aim of the present invention to address one or more of the aforementioned disadvantages associated with the prior art.

### SUMMARY OF THE INVENTION

According to a first aspect of the present invention, there is provided an aircraft fuel dosing system comprising:
a fuel blending device;
a first tank for storing an aircraft fuel;
a second tank for storing a further aircraft fuel or a fuel additive;
a first line configured to transport the aircraft fuel from the first tank to the fuel blending device;
a second line configured to transport the further aircraft fuel or the fuel additive from the second tank to the fuel blending device such that the further aircraft fuel or the fuel additive from the second tank can be combined with the aircraft fuel from the first tank to create a fuel mixture;
a delivery line configured to transport the fuel mixture from fuel blending device to an aircraft fuel consuming device;
at least one sensor configured to monitor at least one parameter associated with the aircraft; and
a dosing control unit configured to receive an output signal from the at least one sensor indicative of the at least one parameter, and being further configured to automatically adjust a composition of the fuel mixture based on the received signal.

Advantageously, the aircraft fuel dosing system according to the claimed invention provides the operator with a closed-loop system for automatically adjusting the composition / ratio of the fuel mixture being delivered to the fuel consuming device without requiring constant human intervention.

For example, the aforementioned aircraft fuel dosing system may enable automatic adjustment of a composition of the fuel mixture during flight to help reduce aircraft emissions, to help ensure completeness of combustion and/or to optimise aircraft performance.

Furthermore, once an optimal fuel mixture has been achieved, the process of monitoring and adjusting the composition of the fuel mixture may be repeated periodically or continuously to ensure that the fuel mixture remains optimised throughout the operation of the aircraft.

Optionally, the at least one sensor may comprise an emission sensor configured to monitor at least one parameter associated with an exhaust gas exhausted from the aircraft fuel consuming device.

Optionally, the at least one parameter may be selected from one or more of: a carbon (soot) content of the exhaust gas; a sulphur content of the exhaust gas; an oxygen (O2) content of the exhaust gas; a nitrous oxide (NOx) content of the exhaust gas, and/or a non-volatile particulate matter (nvPM) content of the exhaust gas.

Optionally, the second tank may contain a sustainable aviation fuel (SAF) or an emission control additive, and the dosing control unit may be configured to adjust the amount of the sustainable aviation fuel (SAF) and/or the amount of the emission control additive introduced into the fuel mixture based on the received signal.

Optionally, the at least one sensor may comprise a first tank sensor configured to monitor at least one parameter associated with the aircraft fuel stored in the first tank.

Optionally, the first tank sensor may be a composition sensor configured to monitor a chemical composition of the aircraft fuel stored in the first tank.

Optionally, the first tank sensor may be a microbial sensor configured to detect the presence of one or more microorganisms (e.g., bacteria) within the aircraft fuel stored in the first tank.

Optionally, the second tank may contain a biocide, and the dosing control unit may be configured to adjust the amount of biocide introduced into the fuel mixture based on the received signal.

Optionally, the first tank sensor may be a viscosity sensor configured to monitor a viscosity of the aircraft fuel contained within the first tank.

Optionally, the second tank may contain a lubricity enhancer, and the dosing control unit may be configured to adjust the amount of lubricity enhancer introduced into the fuel mixture based on the received signal.

Optionally, the at least one sensor may comprise a second tank sensor configured to monitor at least one parameter associated with the aircraft fuel, or the fuel additive, stored in the second tank.

Optionally, the second tank sensor may be a composition sensor configured to monitor a chemical composition of the further aircraft fuel, or the fuel additive, contained within the second tank.

Optionally, aircraft fuel dosing system may further comprise:
a third tank for storing a further aircraft fuel or a further fuel additive; and
a third fuel line configured to transport the further aircraft fuel or the further fuel additive from the third tank to the fuel blending device such that the further aircraft fuel or the further fuel additive from the third tank can be incorporated into the fuel mixture.

Optionally, the at least one sensor may comprise a third tank sensor configured to monitor at least one parameter associated with the aircraft fuel, or the fuel additive, stored in the third tank.

Optionally, the third tank sensor may be a composition sensor configured to monitor a chemical composition of the further aircraft fuel, or the fuel additive, contained within the third tank.

Optionally, the at least one sensor may comprise a fuel blending device sensor configured to monitor at least one parameter associated with the fuel mixture contained in the fuel blending device.

Optionally, the fuel blending device sensor may be a composition sensor configured to monitor a chemical composition of the fuel mixture contained in the fuel blending device.

Optionally, the fuel blending device sensor may be a microbial sensor configured to detect the presence of one or more microorganisms (e.g., bacteria) within the fuel mixture.

Optionally, the second tank may contain a biocide, and the dosing control unit may be configured to adjust the amount of biocide introduced into the fuel mixture based on the received signal.

Optionally, the fuel blending device sensor may be a viscosity sensor configured to monitor a viscosity of the fuel mixture.

Optionally, the second tank may contain a lubricity enhancer, and the dosing control unit may be configured to adjust the amount of lubricity enhancer introduced into the fuel mixture based on the received signal.

Optionally, the fuel blending device sensor may be an electrical sensor configured to monitor the build-up of electrical charge within the fuel blending device.

Optionally, the second tank may contain a static dissipator and the dosing control unit may be configured to adjust the amount of static dissipator introduced into the fuel mixture based on the received signal.

Optionally, the at least one sensor may comprise a delivery line sensor configured to monitor at least one parameter associated with the fuel mixture contained in the delivery line.

Optionally, the delivery line sensor may be a composition sensor configured to monitor a chemical composition of the fuel mixture contained in the delivery line.

Optionally, the at least one sensor may comprise a power rating of less than 100 milliampere (mA).

Optionally, the aircraft fuel dosing system may further comprise a shut-off valve for controlling delivery of the fuel mixture to the aircraft fuel consuming device.

Optionally, the fuel blending device may be located upstream of said shut-off valve.

Optionally, the shut-off valve may comprise:
a valve housing having an internal valve chamber;
a first inlet in fluid communication with the first line for introducing the aircraft fuel into the internal valve chamber;
a second inlet in fluid communication with the second line for introducing the further aircraft fuel or fuel additive into the internal valve chamber; an
an outlet port in fluid communication with the delivery line; and
wherein said internal valve chamber constitutes the fuel blending device of the aircraft fuel dosing system.

Optionally, the shut-off valve may be a low-pressure shut-off valve.

Optionally, the fuel consuming device may be an aircraft engine.

Optionally, the fuel consuming device may be an aircraft auxiliary power unit.

According to a second aspect of the present invention, there is provided an aircraft comprising the aircraft fuel dosing system according to the first aspect of the present invention.

According to a third aspect of the present invention, there is provided a method of automatically adjusting a composition of a fuel mixture on-board an aircraft, said method comprising the steps of:
providing a first tank containing an aircraft fuel;
providing a second tank containing a further aircraft fuel or a fuel additive;
mixing the aircraft fuel or the fuel additive from the second tank with the aircraft fuel from the first tank within a fuel blending device to create a fuel mixture;
monitoring at least one parameter associated with the aircraft;
automatically adjusting a composition of the fuel mixture based on the at least one parameter; and
delivering the fuel mixture to an aircraft fuel consuming device.

Optionally, the method may be performed whilst the aircraft is in flight and/or during ground operations.

Optionally, the at least one parameter may be a parameter associated with the aircraft fuel and/or the fuel additive stored within the first and/or second tanks.

Optionally, the at least one parameter may be a chemical composition of the aircraft fuel stored in the first tank.

Optionally, the at least one parameter may be a chemical composition of the further aircraft fuel, or fuel additive, stored in the second tank.

Optionally, the at least one parameter may be an amount of bacterial contamination contained within the aircraft fuel stored in the first tank.

Optionally, the at least one parameter may be a viscosity of the aircraft fuel stored in the first tank.

Optionally, the second tank may contain a further aircraft fuel, which is different to the aircraft fuel stored in the first tank.

Optionally, the at least one parameter may be a parameter associated with the fuel blending device.

Optionally, the at least parameter may be an electrical charge within the fuel blending device.

Optionally, the at least one parameter may be a parameter associated with the fuel mixture.

Optionally, the at least one parameter may be a chemical composition of the fuel mixture.

Optionally, the at least one parameter may be an amount of bacterial contamination contained within the fuel mixture.

Optionally, the at least one parameter may be a viscosity of the fuel mixture.

Optionally, the at least one parameter may be a parameter associated with an exhaust gas exhausted from the aircraft fuel consuming device.

Optionally, the at least one parameter may be selected from one or more of: a carbon (soot) content of the exhaust gas; a sulphur content of the exhaust gas; an oxygen (O₂) content of the exhaust gas; a nitrous oxide (NOₓ) content of the exhaust gas, and/or a non-volatile particulate matter (nvPM) content of the exhaust gas.

Within the scope of this application, it is expressly intended that the various aspects, embodiments, examples and alternatives set out in the preceding paragraphs, in the claims and/or in the following description and drawings, and in particular the individual features thereof, may be taken independently or in any combination.

That is, all embodiments and/or features of any embodiment can be combined in any way and/or combination, unless such features are incompatible.

The applicant reserves the right to change any originally filed claim or file any new claim accordingly, including the right to amend any originally filed claim to depend from and/or incorporate any feature of any other claim although not originally claimed in that manner.

### BRIEF DESCRIPTION OF THE DRAWINGS

Embodiments of the invention will now be described with reference to the accompanying drawings, in which:
Figure 1 is a schematic front view of an aircraft according to an embodiment of the present invention;
Figure 2 is a schematic plan view of the aircraft illustrated in Figure 1;
Figure 3 is a schematic illustration of an aircraft fuel dosing system according to an embodiment of the present invention;
Figure 4A is a schematic illustration of a fuel blending device which may be incorporated into the aircraft fuel dosing system illustrated in Figure 3;
Figure 4B is a schematic illustration of an alternative fuel blending device which may be incorporated into the aircraft fuel dosing system illustrated in Figure 3;
Figure 5 is a schematic plan view of an aircraft incorporating an aircraft fuel dosing system according to another embodiment of the present invention;
Figure 6A is a schematic illustration of a fuel blending device which may be incorporated into the aircraft fuel dosing system illustrated in Figure 5;
Figure 6B is a schematic illustration of an alternative fuel blending device which may be incorporated into the aircraft fuel dosing system illustrated in Figure 5;
Figure 7A is a schematic illustration of the aircraft fuel dosing system illustrated in Figure 5;
Figure 7B is a schematic illustration of an aircraft fuel dosing system according to another embodiment of the present invention; and
Figure 8 is a schematic illustration of an aircraft fuel dosing system according to a further embodiment of the present invention.

### DETAILED DESCRIPTION OF EMBODIMENT(S)

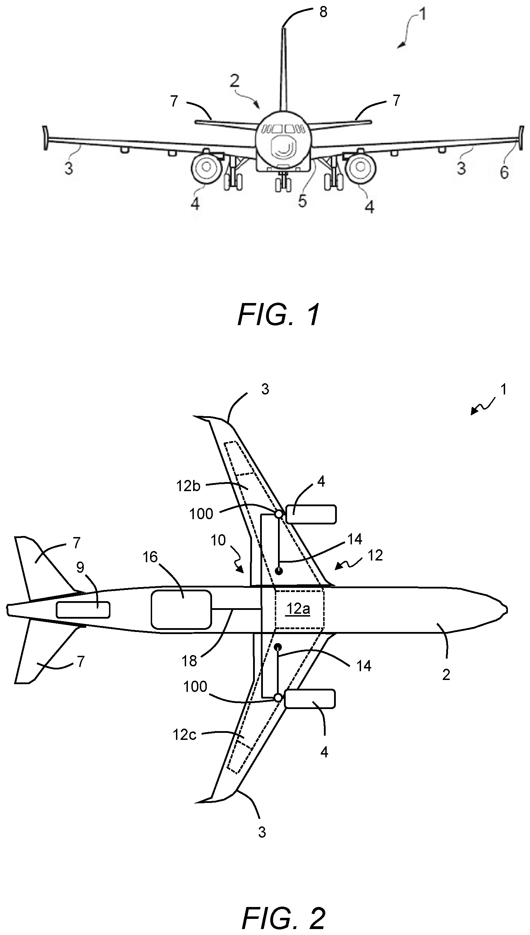

Figure 1 shows an aircraft 1 according to an embodiment of the present invention.

In the embodiment illustrated in Figure 1, the aircraft 1 is a typical jet passenger transport aircraft but the invention is applicable to a wide variety of fixed wing aircraft types, including commercial, military, passenger, cargo, jet, propeller, general aviation, etc.

The aircraft 1 has a fuselage 2 and two wings 3 extending from either side of the fuselage 2. Each wing 3 extends in a spanwise direction from a wing root 5 where the wing 3 meets the fuselage 2 to a wing tip 6. The aircraft 1 also comprises an empennage (or tail assembly) which includes starboard and port horizontal stabilisers 7 and a vertical stabiliser 8.

Engines 4 are located on the underside of each wing 3. Figure 1 shows a single engine 4 on each wing, however the number and location of the engines may vary depending on the type of aircraft.

In the embodiment illustrated in Figure 1, each wing 3 is a dihedral wing, so the wings 3 are angled upwardly as they extends towards the wing tip 6. However, it shall be appreciated that the present invention may also be used in aircraft having anhedral wings or any other suitable wing design.

The aircraft 1 also includes an auxiliary power unit 9 (often referred to as an "APU") which is mounted in the fuselage 2 proximate to the tail assembly (or empennage).

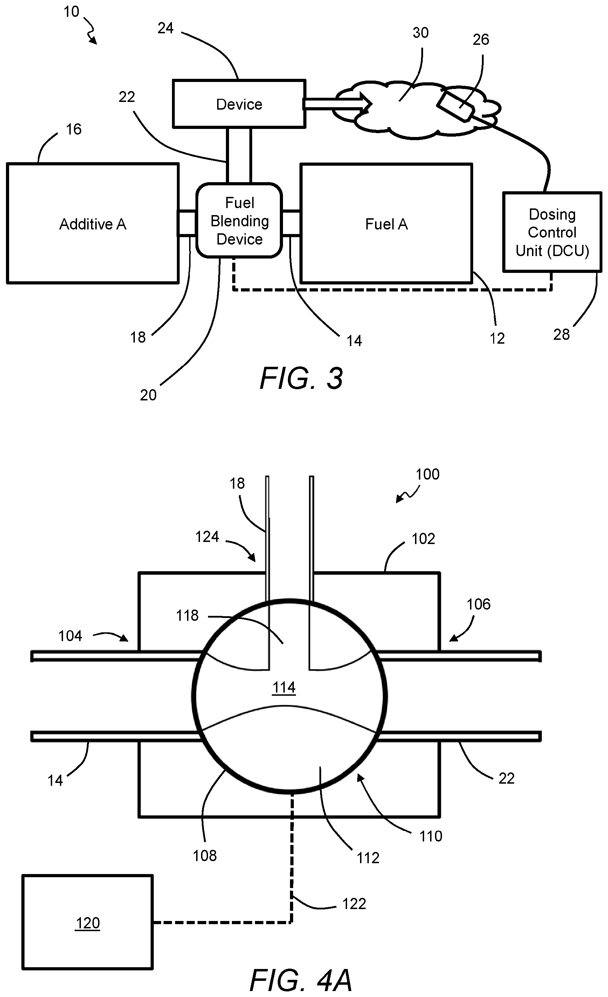

Figures 2 and 3 show a schematic representation of an aircraft fuel dosing system 10 according to an embodiment of the present invention.

The aircraft fuel dosing system 10 includes a first tank 12, a first line 14 in fluid communication with the first tank 12, a second tank 16, a second line 18 in fluid communication with the second tank 16, a fuel blending device 20 in fluid communication with the first 14 and second 18 lines and a delivery line 22 in fluid communication with the fuel blending device 20 for delivering a fuel mixture from the fuel blending device 20 to an aircraft fuel consuming device 24.

In the illustrated embodiment, the aircraft fuel consuming device 24 is an engine 4 of the aircraft 1. However, it shall be appreciated that in other embodiments, the delivery line 22 of the aircraft fuel dosing system 10 may be arranged to deliver the fuel mixture to other types of fuel consuming device 24 which may be present on-board the aircraft 1, such as the auxiliary power unit (APU) 9.

The first tank 12 is configured for containing and storing an aircraft fuel, such as a synthetic fuel, a sustainable aviation fuel (SAF), a kerosene-based fuel, such as Jet A1, or a combination thereof.

In the embodiment illustrated in Figure 2, the first tank 12 is made up of a plurality of sub-tanks 12a, 12b, 12c. The sub-tanks shown in Figure 2 comprise a centre tank 12a provided in the fuselage 2; a first wing tank 12b provided in the port wing 3; and a second wing tank 12c provided in the starboard wing. However, it shall be appreciated that in other embodiments, the first tank 12 may be provided as a single tank within the wing 3 or fuselage 2 structure.

The second tank 16 is configured for containing and storing a further aircraft fuel, which is different to the aircraft fuel stored in the first tank 12, or a fuel additive.

In some embodiments, the further aircraft fuel may be a liquid that has a different chemical structure to that of the aircraft fuel stored in the first tank 12. For example, the further aircraft fuel may be a sustainable aviation fuel (SAF) that has a higher hydrogen count than Jet A1 or may be a blend of suitable biofuels.

Alternatively, the fuel additive may be a biocide, a metal deactivator, a lubricity enhancer, a detergent, a solvent, an emission control additive, an anti-static additive, or any other suitable fuel additive composition.

It shall be appreciated that the fuel additive may be provided as a liquid, a suspended powder in liquid, a solvent, a surfactant, a gaseous mixture, a dissolved mixture in an aviation fuel or any combination of the above.

In the embodiment illustrated in Figure 2, the second tank 16 is provided as a single tank within the fuselage 2 structure of the aircraft 1. However, it shall be appreciated that in other embodiments, the second tank 16 may be made up of a plurality of sub-tanks and/or may be provided at other locations about the aircraft 1.

The first 12 and second 16 tanks are loaded with an aircraft fuel (or a fuel additive) before the aircraft 1 takes flight. The first 14 and second 18 lines subsequently deliver the aircraft fuel from the first tank 12 and the further aircraft fuel (or fuel additive) from the second tank 16 to the fuel blending device 20 such that the aircraft fuel from the first tank 12 can be combined with the further aircraft fuel (or fuel additive) from the second tank 16 to create a fuel mixture which is subsequently delivered to the fuel consuming device 24 via the delivery line 22.

It shall be appreciated that the first line 14 may be any suitable conduit that is configured to transport the aircraft fuel contained in the first tank 12. Similarly, it shall be appreciated that the second line 18 may be any suitable conduit that is configured to transport the further aircraft fuel (or the fuel additive) contained in the second tank 16.

It shall also be appreciated that storing the further aircraft fuel (or fuel additive) in a separate tank reduces the risk of the contents "going off" or settling in the main fuel tank, thereby achieving improved storage stability.

The aircraft fuel dosing system 10 may further comprise one or more shut-off valves 100 located along the delivery line 22 for controlling the delivery of the fuel mixture from the fuel blending device 20 to the fuel consuming device 24.

In the embodiment illustrated in Figure 2, the aircraft fuel dosing system 10 comprises a pair of low-pressure shut-off valves (often referred to as "LPSOVs") which are located at the leading edges of the port and starboard wings 3 respectively.

However, it shall be appreciated that in other embodiments, the one or more shut-off valves 100 may be of a different type. For example, in alternate arrangements, the shut-off valves may be high-pressure shut-off valves (often referred to as "HPSOVs") located downstream of the low-pressure shut-off valves 100 and upstream of the fuel consuming device 24.

Figure 4A shows a schematic representation of a respective one of the low-pressure shut-off valves 100 illustrated in Figure 2.

As shown in Figure 4A, the shut-off valve 100 comprises a valve housing 102 having a first inlet port 104, a second inlet port 124, an outlet port 106 and an internal valve chamber 108 which together form a fluid flow path across the valve housing 102.

The first inlet port 104 is in fluid communication with the first line 14 of the aircraft fuel dosing system 10 such that the aircraft fuel from the first tank 12 can be delivered through the first line 14 and into the internal valve chamber 108.

The second inlet port 124 is in fluid communication with the second line 18 of the aircraft fuel dosing system 10 such that the further aircraft fuel (or the fuel additive) from the second tank 16 can be delivered through the second line 18 and into the internal valve chamber 108.

The outlet port 106 is in fluid communication with the delivery line 22 such that the aircraft fuel, or the fuel mixture, can be conveyed from the internal valve chamber 108 to the fuel consuming device 24 when the shut-off valve 100 is in a first, open, position.

A valve member 110 is housed within the internal valve chamber 108 of the valve housing 102, the valve member 110 being positioned in the fluid flow path.

In the illustrated embodiment, the shut-off valve 100 is a rotary ball valve and hence the valve member 110 has a substantially spherical body portion 112 having a first channel 114 extending therethrough. However, it shall be appreciated that in other embodiments, the shut-off valve 100 may be another kind of a rotary valve. It shall also be appreciated that in further embodiments, the shut-off valve may be another suitable valve type, such as a linear motion valve or a pivoting valve.

The first channel 114 of the valve member 110 is configured to align with the first inlet port 104 and the outlet port 106 of the valve housing 102 when the valve member 110 is in the first, open, position to enable the aircraft fuel from the first tank 12 to travel along the fluid flow path.

The body portion 112 of the valve member 110 also comprises a second channel 118 extending between the first channel 114 and an outer surface of the body portion 112.

The second channel 118 of the valve member 110 is orientated substantially perpendicular to the first channel 114 and is configured to align with the second inlet port 124 when the valve member 110 is in the first, open, position to enable the further aircraft fuel (or the fuel additive) to travel along the second channel 118 and into the first channel 114 where it can be combined with the aircraft fuel from the first tank 12 to create the fuel mixture.

As such, it shall be appreciated that in the embodiment illustrated in Figure 4A, the internal valve chamber 108 of the shut-off valve 100 constitutes the fuel blending device 20. In other words, the fuel blending device 20 is incorporated into the low-pressure shut-off valve 100.

The internal valve chamber 108 has a shape corresponding to that of the valve member 110, with the diameter of the internal valve chamber 108 being slightly larger than that of the valve member 110 so as to allow the valve member 110 to freely rotate within the internal valve chamber 108.

The valve member 110 is attached to a valve member drive shaft 122 which extends from the valve member 110 and passes through the valve housing 102.

The shut-off valve 100 also comprises a valve actuator 120 which is configured to apply an actuating force to the valve member drive shaft 122 which in turn transfers the actuating force onto the valve member 110 thereby causing the valve member 110 to rotate.

The valve actuator 120 is operable to move the valve member 110 between the first, open, position in which fluid is able to freely flow along the fluid flow path and a second, closed, position in which the fluid flow path is blocked by the valve member body 112 such that fluid is not able to flow along the fluid flow path.

As such, by actuating the valve member 110 from the first, open, position to the second, closed, position, the delivery of the fuel mixture from the first 12 and second 16 tanks to the fuel consuming device 24 can be controlled.

For example, during normal operation of the aircraft 1, the valve member 110 may be placed in the first, open, position to enable the fuel mixture to be delivered to the fuel consuming device 24.

However, in the event of an emergency engine shutdown or during a scheduled maintenance activity, the valve member 110 may be placed in the second, closed, position to substantially restrict (or "shut-off) the flow of the fuel mixture to the fuel consuming device 24.

The valve actuator 120 is also operable to move the valve member 110 into one or more further positions in which the flow of the aircraft fuel from the first inlet port 104 into the internal valve chamber 108 and/or the flow of the further aircraft fuel (or fuel additive) from the second inlet port 124 into the internal valve chamber 108 is partially restricted.

For example, when the valve member 110 is rotated by an angular distance of between 1 and 89 degrees (for example by approximately 45 degrees) from the first, open, position to a further, third position, the first channel 114 of the valve member 110 will no longer be fully aligned with the first inlet port 104 and hence the flow of the aircraft fuel from the first inlet port 104 into the first channel 114 of the valve member 110 will be partially restricted by the valve member body 112.

As such, whilst the aircraft fuel is still able to flow from the first inlet port 104 to the outlet port 106 when the valve member 110 is rotated to the further, third position, the volume of aircraft fuel flowing along the fluid flow path will be reduced when compared to the volume of aircraft fuel flowing along the fluid flow path when the valve member 110 is in the first, fully open, position.

Similarly, when the valve member 110 is rotated into the further, third position, the second channel 118 of the valve member 110 will also no longer be fully aligned with the second inlet port 124 and hence the flow of the further aircraft fuel (or the fuel additive) from the second inlet port 124 into the second channel 118 will also be partially restricted by the valve member body 112.

As such, whilst the further aircraft fuel (or the fuel additive) is still able to flow from the second inlet port 124 into the internal valve chamber 108 when the valve member 110 is rotated to the further, third position, the volume of the further aircraft fuel (or the fuel additive) flowing from the second inlet port 124 into the internal valve chamber 108 will also be reduced when compared to the volume of the further aircraft fuel (or the fuel additive) flowing from the second inlet port 124 to the internal valve chamber 108 when the valve member 110 is in the first, fully open, position.

As shall be described in greater detail later within the present application, it shall be appreciated that by adjusting the position of the valve member 110, the aircraft fuel dosing system 10 is able to adjust the amount of aircraft fuel and/or fuel additive being delivered into the internal valve chamber 108, and hence, by actuating the valve member 110 between the first and third positions, the chemical composition of the fuel mixture created within the internal valve chamber 108 can be tailored (or adjusted) during the operation of the aircraft.

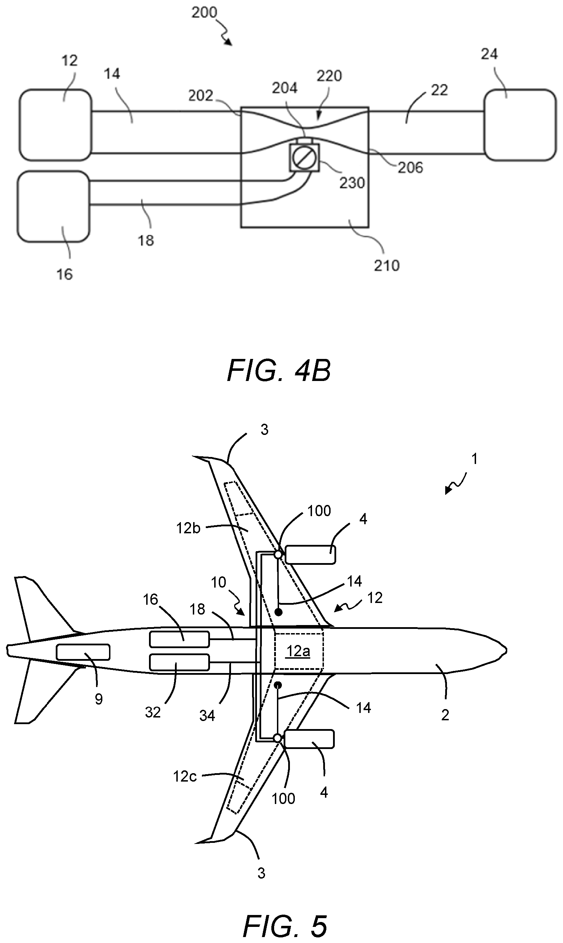

Figure 4B shows a schematic representation of an alternative fuel blending device 200 which may be incorporated into the aircraft fuel dosing system 10 illustrated in Figure 3.

It shall be appreciated that the fuel blending device 200 illustrated in Figure 4B is designed to be located upstream of the low-pressure shut-off valve 100 and hence, unlike the embodiment illustrated in Figure 4A, the fuel blending device 200 illustrated in Figure 4B is provided separately from the low-pressure shut-off valve 100.

As shown in Figure 4B, the fuel blending device 200 has a first inlet 202 that is in fluid communication with the first line 14, a second inlet 204 that is in fluid communication with the second line 18 and an outlet 206 that is in fluid communication with the delivery line 22.

A chamber 210 is positioned in between the first inlet 202, the second inlet 204 and the outlet 206. The fuel blending device 200 therefore has a flow path between the first inlet 202, the second inlet 204 and the outlet 206.

The fuel blending device 200 also includes a pressure reduction feature 220 provided within the chamber 210. The pressure reduction feature 220 is configured to create a local velocity increase within the chamber 210, which results in a pressure reduction that is able to draw the further aircraft fuel (or fuel additive) from the second line 18 into the chamber 210 where it can be combined with the aircraft fuel from the first tank 12 to create the fuel mixture.

It shall be appreciated that the local velocity increase created by the pressure reduction feature 220 also helps to mix the aircraft fuel from the first tank 12 with the aircraft fuel (or the fuel additive) from the second tank 16.

In the example shown in Figure 4B, the pressure reduction feature 220 is a venturi device. However, it will be understood that any arrangement of pressure reduction feature 220 may be employed in the fuel blending device 200 (i.e. any arrangement that would alter the pressure along the flow path from the first inlet 202, second inlet 204 and the outlet 206), such as orifice plates, control valves and/or flow restrictors.

Referring still to Figure 4B, the fuel blending device 200 further includes a control valve 230 arranged on the second line 18. However, it shall be appreciated that in other embodiments, the control valve 230 may instead be arranged on the first line 14, or in further alternatives, a pair of control valves 230 may be provided on both the first 14 and second 18 lines.

The control valve 230 is operable to move between a first, open position and a second, closed position. In the open position, the aircraft fuel (or the fuel additive) from the second line 18 is free to flow through the second inlet 204 and into the chamber 210.

In the closed position, the aircraft fuel (or the fuel additive) from the second line 18 is prevented from flowing through the second inlet 204 and into the chamber 210 of the fuel blending device 200.

As such, by actuating the control valve 230 between the first and second positions, the chemical composition of the fuel mixture can be tailored (or adjusted) during the operation of the aircraft.

Referring back to Figure 3, the aircraft fuel dosing system 10 further comprises at least one sensor 26 and a dosing control unit 28 which is configured to automatically adjust a composition of the fuel mixture as shall be described in detail below.

The at least one sensor 26 is configured to monitor one or more parameters associated with the aircraft 1 and outputs a signal which is indicative of the one or more parameters associated with aircraft 1 to the dosing control unit 28.

It shall be appreciated that the at least one sensor 26 may communicate with the dosing control unit 28 via wired or wireless communication means.

In the illustrated embodiment, the at least one sensor 26 is an emission sensor configured to monitor at least one parameter associated with an exhaust gas 30 of the fuel consuming device 24.

For example, the at least one sensor 26 may comprise a visual sensor (e.g., a camera) for monitoring a carbon (or soot) content of the exhaust gas 30, a nitrous oxide (or NO_{X}) sensor for monitoring a nitrous oxide (NO_{X}) content of the exhaust gas 30 and/or an oxygen (or lambda) sensor for monitoring an oxygen (O₂) content of the exhaust gas 30.

It shall be appreciated that the aforementioned types of emission sensor are well-known to a person skilled in the art and so, for the sake of conciseness, shall not be described in detail within this application.

The at least one sensor 26 may also be configured to monitor other emission parameters, such as a sulphur content of the exhaust gas 30 and/or a non-volatile particulate matter (nvPM) content of the exhaust gas 30.

The dosing control unit 28 may comprise one or more processors which may be configured to compare the output signal received from the at least one sensor 26 with a desired reference threshold or set point.

In the event that the output signal received from the at least one sensor 26 falls outside of the desired reference threshold (or falls above or below the aforementioned set point), the one or more processors may be further configured to generate an error signal and determine a necessary control action in order to minimise the error signal.

As such, upon determining that an emission content (e.g., a NO_{X} content) of the exhaust gas 30 falls above a given emission threshold, the dosing control unit 28 may generate or adjust a control action for operating the valve actuator 120 so as to adjust a position of the valve member 110 or control valve 230.

As set out above, it shall be appreciated that adjusting the position of the valve member 110 or control valve 230 will alter the rate at which the aircraft fuel and/or fuel additive enters the fuel blending device 20, which, in turn, will alter the chemical composition of the fuel mixture generated therein.

For example, in some embodiments, the second tank may contain a sustainable aviation fuel (SAF) and hence, upon determining that an emission content of the exhaust gas 30 is above a pre-determined emission threshold, the dosing control unit 28 may control the valve actuator 120 so as to increase the amount of sustainable aviation fuel (SAF) being introduced into the fuel mixture until the emission content of the exhaust gas 30 has been brought back within the pre-determined emission threshold.

As such, the aircraft fuel dosing system 10 according to the claimed invention provides the operator with a closed-loop system for automatically adjusting the composition / ratio of the fuel mixture being delivered to the fuel consuming device 24 without requiring constant human intervention.

Furthermore, once an optimal fuel mixture has been achieved, the process of monitoring the emission content of the fuel consuming device 24 and adjusting the composition of the fuel mixture accordingly may be repeated periodically or continuously to ensure that the fuel mixture remains optimised throughout the operation of the aircraft.

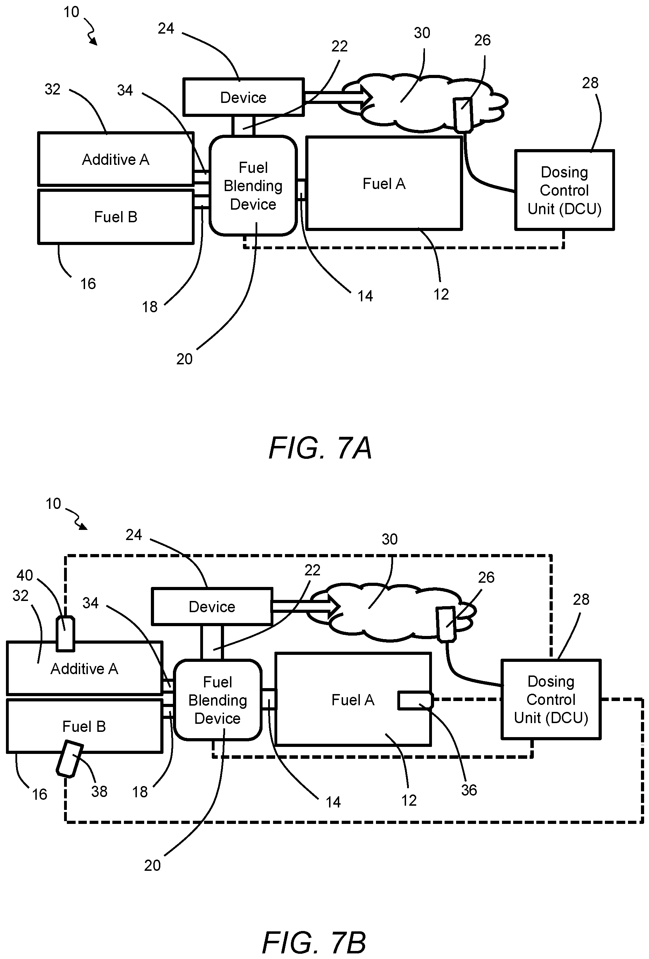

Figures 5 and 7A show a schematic representation of an aircraft fuel dosing system 10 according to an alternative embodiment of the present invention.

It shall be appreciated that the aircraft fuel dosing system 10 depicted in Figures 5 and 7A has many features in common with the aircraft fuel dosing system 10 depicted in Figures 2 and 3 and so, for the sake of conciseness, only the differences shall be described herein. Like components have been denoted by corresponding reference numerals.

Notably, the aircraft fuel dosing system 10 illustrated in Figure 5 further comprises a third tank 32 and a third line 34 in fluid communication with the third tank 32.

The third tank 32 is configured for containing and storing a fuel additive. It shall be appreciated that in embodiments in which the second tank 16 also contains a fuel additive, the further fuel additive stored in the third tank 32 will be different to the fuel additive stored in the second tank 16.

In some embodiments, the fuel additive may be a liquid that has a different chemical structure to that of the aircraft fuel stored in the first tank 12, and to that of the further aircraft fuel (or fuel additive) stored in the second tank 16.

For example, the fuel additive may be a bio-additive for combining with the sustainable aviation fuel (SAF) and the "Jet A1" aircraft fuel contained within the first 12 and second 16 tanks respectively.

Alternatively, the fuel additive may be a biocide, a metal deactivator, a lubricity enhancer, a detergent, a solvent, an emission control additive, an anti-static additive, or any other suitable fuel additive composition.

It shall be appreciated that the fuel additive may be provided as a liquid, a suspended powder in liquid, a solvent, a surfactant, a gaseous mixture, a dissolved mixture in an aviation fuel or any combination of the above.

In the embodiment illustrated in Figure 5, the third tank 32 is provided as a single tank within the fuselage 2 structure of the aircraft 1. However, it shall be appreciated that in other embodiments, the third tank 32 may be made up of a plurality of sub-tanks and/or may be provided at other locations about the aircraft 1.

The third tank 32 is loaded with the fuel additive before the aircraft 1 takes flight. The third line 34 subsequently delivers the fuel additive from the third tank 32 to the fuel blending device 20 such that the fuel additive from the third tank 32 can be incorporated into the fuel mixture which is subsequently delivered to the fuel consuming device 24 via the delivery line 22.

It shall be appreciated that the third line 34 may be any suitable conduit that is configured to transport the fuel additive contained in the third tank 32.

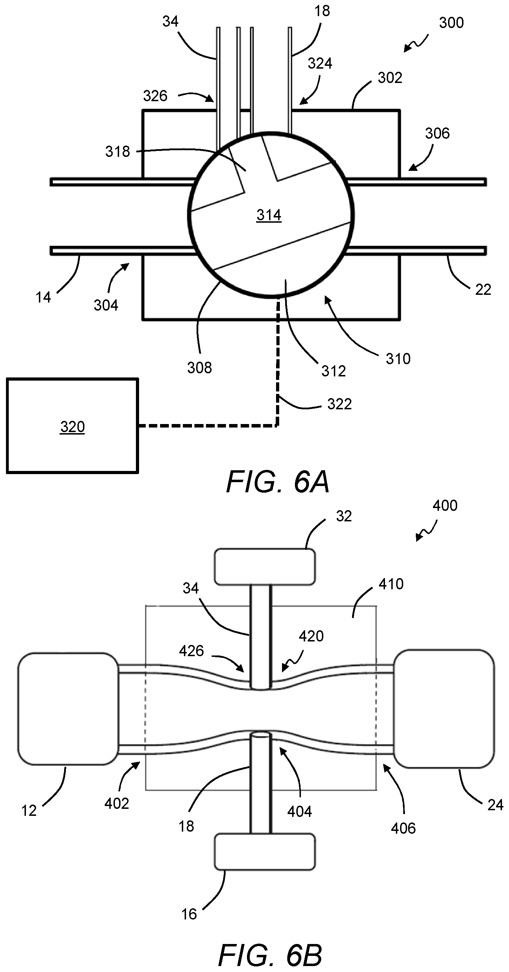

Figure 6A shows a schematic representation of a fuel blending device 300 which may be incorporated into the aircraft fuel dosing system 10 illustrated in Figure 5.

It shall be appreciated that the fuel blending device 300 depicted in Figure 6A has many features in common with the shut-off valve 100 depicted in Figure 4A and so, for the sake of conciseness, only the differences shall be described herein. Like components have been denoted by corresponding reference numerals with the prefix "3".

As shown in Figure 6A, the valve housing 302 of the fuel blending device 300 further comprises a third inlet port 326 which is in fluid communication with the third line 34 of the aircraft fuel dosing system 10 such that the fuel additive from the third tank 32 can be delivered through the third line 34 and into the internal valve chamber 308.

In the illustrated embodiment, the third inlet port 326 is spaced apart from the first inlet port 304 by an angular distance of approximately 90 degrees such that the third inlet port 326 is positioned adjacent to the second inlet port 324.

As such, when the valve member 310 is in the first, open, position, the third inlet port 326 is not able to align with the second channel 318 provided in the valve member 310 and hence the passage of the fuel additive from the third inlet port 326 into the internal valve chamber 308 is substantially restricted (or "blocked") by the valve member body 312.

As such, when the valve member 310 is in the first position, the fuel additive from the third tank 32 is not able to flow into the first channel 314 and hence only a mixture of the aircraft fuel from the first tank 12 and the further aircraft fuel (or the fuel additive) from the second tank 16 is delivered to the fuel consuming device 24.

However, as shown in Figure 6A, when the valve member 310 is rotated by an angular distance of between 1 and 89 degrees (for example by approximately 45 degrees) from the first, open, position to a further, third position, the first channel 314 of the valve member 310 remains partially aligned with the first inlet port 304 and the outlet port 306 of the valve housing 302 and hence the aircraft fuel from the first tank 12 is able to flow from the first inlet port 304 to the outlet port 306.

However, when the valve member 310 is rotated into the further, third position, the second channel 318 of the valve member 310 also becomes partially aligned with both the second inlet port 324 and the third inlet port 326 and hence both the further aircraft fuel (or the fuel additive) from the second tank 16 and the fuel additive from the third tank 32 are able to flow into the second channel 318 (via the second 324 and third 326 inlet ports) and into the first channel 314 where they can be combined with the aircraft fuel from the first tank 12 to create, or enhance, the chemical composition of the fuel mixture.

As such, it shall be appreciated that by adjusting the position of the valve member 310, the aircraft fuel dosing system 10 is able to adjust the amount of the fuel additive being delivered into the internal valve chamber 308 via the third inlet port 326, and hence, by actuating the valve member 310 between the first and third positions, the chemical composition of the fuel mixture can be further tailored (or adjusted) during the operation of the aircraft.

Figure 6B shows a schematic representation of an alternative fuel blending device 400 which may be incorporated into the aircraft fuel dosing system 10 illustrated in Figure 5.

It shall be appreciated that the fuel blending device 400 depicted in Figure 6B has many features in common with the fuel blending device 200 depicted in Figure 4B and so, for the sake of conciseness, only the differences shall be described herein. Like components have been denoted by corresponding reference numerals with the prefix "4".

As shown in Figure 6B, the fuel blending device 400 further comprises a third inlet port 426 which is in fluid communication with the third line 34 of the aircraft fuel dosing system 10 such that the fuel additive from the third tank 32 can be delivered through the third line 34 and into the chamber 410.

As such, the chamber 410 of the fuel blending device 400 illustrated in Figure 6B is positioned in between the first inlet port 402, the second inlet port 404, the third inlet port 426 and the outlet 406.

Although not shown in Figure 6B, it shall also be appreciated that the fuel blending device 400 may comprise a further control valve operable to move between a first, open position and a second, closed position.

In the open position, the fuel additive from the third line 34 may be free to flow through the third inlet 426 and into the chamber 410. In the closed position, the fuel additive from the third line 34 may be prevented from flowing through the third inlet 426 and into the chamber 410 of the fuel blending device 400.

As such, by actuating the further control valve between the first and second positions, the amount of fuel additive introduced into the chamber 410 can be tailored (or adjusted) during the operation of the aircraft.

Referring now to Figure 7A, in some embodiments, the third tank 32 may contain an emission control additive and hence, upon determining that an emission content of the exhaust gas 30 is above a pre-determined emission threshold, the dosing control unit 28 may send a control action (or control signal) to the valve actuator 320 to increase the amount of the emission control additive being incorporated into the fuel mixture to help reduce aircraft emissions and/or to help ensure completeness of combustion until the emission content of the exhaust gas 30 has been brought back within the pre-determined emission threshold.

Furthermore, once an optimal fuel mixture has been achieved, the process of monitoring the emission content of the fuel consuming device 24 and adjusting the composition of the fuel mixture accordingly may be repeated periodically or continuously to ensure that the fuel mixture remains optimised throughout the operation of the aircraft.

In some embodiments, the aircraft fuel dosing system 10 may also comprise one or more further sensors in addition to, or in place of, the emission sensor 26 described above.

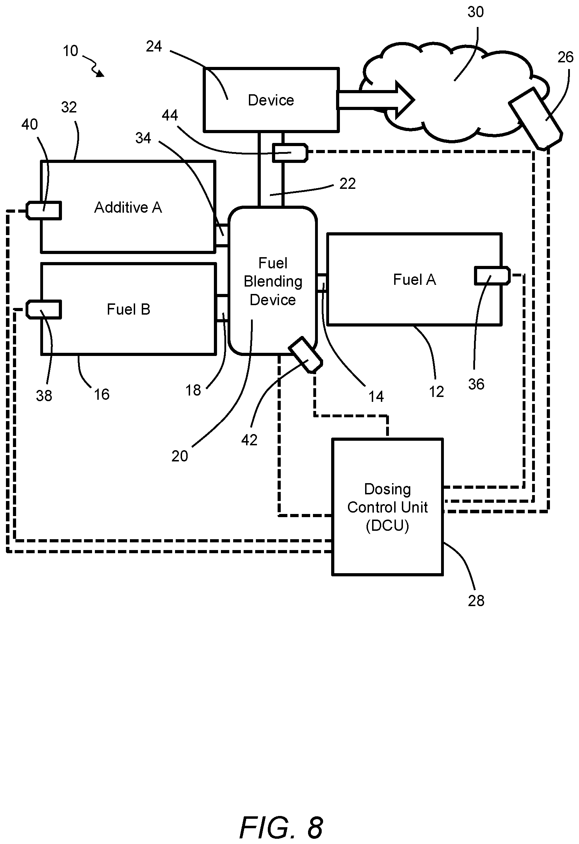

The one or more further sensors shall now be described in detail with reference to Figure 7B and Figure 8.

It shall be appreciated that the one or more sensors incorporated into the aircraft fuel dosing system 10 will typically be "low power" sensors having a power rating of less than 100 milliampere (mA) to reduce the risk of ignition, which is of particular importance for sensors deployed in close proximity to aircraft fuel systems.

Figure 7B shows a schematic representation of an aircraft fuel dosing system 10 according to another embodiment of the present invention.

It shall be appreciated that the aircraft fuel dosing system 10 depicted in Figure 7B has many features in common with the aircraft fuel dosing system 10 depicted in Figure 7A and so, for the sake of conciseness, only the differences shall be described herein. Like components have been denoted by corresponding reference numerals.

As shown in Figure 7B, in addition to the emission sensor 26 (described above with reference to Figure 3), the aircraft fuel dosing system 10 also comprises a first tank sensor 36 which is located within the first tank 12 for monitoring one or more parameters associated with the aircraft fuel contained therein.

In the illustrated embodiment, the first tank sensor 36 is a composition sensor configured to monitor a chemical composition of the aircraft fuel stored in the first tank 12 and subsequently output a signal which is indicative of said composition to the dosing control unit 28.

It shall be appreciated that the first tank sensor 36 may communicate with the dosing control unit 28 via wired or wireless communication means.

In some embodiments, the dosing control unit 28 may comprise one or more processors configured to determine (or verify) the composition of the aircraft fuel stored in the first tank 12 based on the output signal received from the first tank sensor 36.

The dosing control unit 28 may be further configured to automatically adjust the amount of the aircraft fuel incorporated into the fuel mixture based on the chemical composition of the aircraft fuel contained in the first tank 12.

For example, it shall be appreciated that when the aircraft 1 is refuelled, the contents of the first tank 12 will naturally become mixed by the addition of the new fuel. For example, if the aircraft 1 fills up in Dubai, the composition of the aircraft fuel in this location may be slightly different to that at Heathrow due to differences in supplier etc.

The aforementioned dosing control unit 28 is able to account for such variations in fuel composition so as to ensure that a desired fuel ratio is being delivered to the fuel consuming device 24.

The dosing control unit 28 may also be configured to activate the shut-off valve 300 so as to shut-off the supply of the fuel mixture to the fuel consuming device 24 in the event that the one or more processors determine that an incorrect fuel has been added to the first tank 12.

In other embodiments, the first tank sensor 36 may be configured to monitor other parameters associated with the aircraft fuel stored in the first tank 12.

For example, in the United Kingdom, in order for an aircraft fuel to be classified as "Jet Al", the aircraft fuel must fall within a particular set of specifications and/or standards (such as DEF-STAN-91-091).

The aforementioned standards may prescribe various thresholds which must be met by the aircraft fuel such as a maximum acceptable contamination threshold; a density threshold; a viscosity (or lubricity) threshold; an aromatic threshold; a sulphur threshold; an autoignition temperature threshold and/or a synthetic component threshold.

For example, in some embodiments, the first tank sensor 36 may be configured to monitor an autoignition temperature of the aircraft fuel stored in the first tank 12 and subsequently output a signal which is indicative of said autoignition temperature to the dosing control unit 28.

In some embodiments, the dosing control unit 28 may comprise one or more processors which may be configured to compare the output signal received from the first tank sensor 36 with an autoignition temperature threshold for the aircraft fuel obtained from an aircraft fuel standard (such as DEF-STAN-91-091).

In the event that the output signal received from the first tank sensor 36 falls outside the aforementioned autoignition temperature threshold, the one or more processors may be further configured to generate an error signal and determine a necessary control action in order to minimize the error signal.

For example, upon determining that the autoignition temperature of the aircraft fuel contained within the first tank 12 falls outside of a pre-determined autoignition temperature threshold, the dosing control unit 28 may send a control action (or control signal) to the valve actuator 320 so as to adjust the chemical composition of the fuel mixture to ensure that the fuel mixture contained within the fuel blending device 20 adheres to the required standards.

In other examples, the first tank sensor 36 may be a viscosity sensor configured to monitor a viscosity of the aircraft fuel stored in the first tank 12 and subsequently output a signal which is indicative of said viscosity to the dosing control unit 28.

The dosing control unit 28 may comprise one or more processors which may be configured to compare the output signal received from the first tank sensor 36 with a viscosity threshold for the aircraft fuel obtained from the DEF-STAN-91-091 standard or another aircraft fuel standard.

In the event that the output signal received from the first tank sensor 36 falls outside the aforementioned viscosity threshold, the one or more processors may be further configured to generate an error signal and determine a necessary control action in order to minimize the error signal.

For example, in some embodiments, the second 16 and/or third 32 tanks may contain a lubricity enhancer. As such, upon determining that the viscosity (or lubricity) of the aircraft fuel contained within the first tank 12 falls outside a pre-determined viscosity threshold, the dosing control unit 28 may send a control action (or control signal) to the valve actuator 320 so as to increase or decrease the amount of lubricity enhancer being supplied to the fuel blending device 20 to help ensure that the fuel mixture being supplied to the fuel consuming device 24 exhibits a viscosity (or lubricity) that is within the pre-determined viscosity threshold.

In other embodiments, the first tank sensor 36 may be a microbial sensor configured to monitor the presence of one or more microorganisms (e.g., bacteria) within the aircraft fuel stored in the first tank 12 and subsequently output a signal which is indicative of said microbial levels to the dosing control unit 28.

The dosing control unit 28 may comprise one or more processors which may be configured to compare the output signal received from the first tank sensor 36 with a pre-determined microbial level threshold for the aircraft fuel.

In the event that the output signal received from the first tank sensor 36 falls outside the aforementioned microbial level threshold, the one or more processors may be further configured to generate an error signal and determine a necessary control action in order to minimize the error signal.

For example, in some embodiments, the second 16 and/or third 32 tanks may contain a biocide. As such, upon determining that the microbial level of the aircraft fuel contained within the first tank 12 falls outside a pre-determined microbial level threshold, the dosing control unit 28 may send a control action (or control signal) to the valve actuator 320 so as to increase the amount of biocide being supplied to the fuel blending device 20 to help ensure that the microbial level of the fuel mixture being supplied to the fuel consuming device 24 falls within below the pre-determined microbial level.

It shall also be appreciated that in some embodiments, the first tank 12 may comprise a plurality of sensors configured to monitor a plurality of parameters associated with the aircraft fuel stored therein.

Referring still to Figure 7B, the aircraft fuel dosing system 10 may also comprise a second tank sensor 38 located in the second tank 16 for monitoring one or more parameters associated with the aircraft fuel (or the fuel additive) contained therein.

In the illustrated embodiment, the second tank sensor 38 is a composition sensor configured to monitor a chemical composition of the aircraft fuel stored in the second tank 16 and subsequently output a signal which is indicative of said composition to the dosing control unit 28.

It shall be appreciated that the second tank sensor 38 may communicate with the dosing control unit 28 via wired or wireless communication means.

In some embodiments, the dosing control unit 28 may comprise one or more processors configured to determine (or verify) the composition of the aircraft fuel (or the fuel additive) stored in the second tank 16 based on the output signal received from the second tank sensor 38.

The dosing control unit 28 may be further configured adjust the amount of the aircraft fuel (or the fuel additive) incorporated into the fuel mixture based on the chemical composition of the aircraft fuel contained in the second tank 16 to account for variations in fuel composition so as to ensure that a desired fuel ratio is being delivered to the fuel consuming device 24.

The dosing control unit 28 may also be configured to activate the shut-off valve 300 so as to shut-off the supply of the fuel mixture to the fuel consuming device 24 in the event that the one or more processors determine that an incorrect fuel (or fuel additive) has been added to the second tank 16.

In other embodiments, the dosing control unit 28 may be further configured to prioritise the use of one of the aircraft fuels stored in the first 12 and second 16 tanks respectively based on the determined compositions.

For example, the first tank 12 may contain a high-performance aircraft fuel (such as Jet A1), and the second tank 16 may contain a sustainable aviation fuel (such as SAF).

In scenarios in which the user wishes to suppress contrail formation, enhance local air quality or reduce cost during a given flight phase, the dosing control unit 28 may be configured to prioritise the supply of the sustainable aviation fuel (SAF) to the fuel consuming device 24 and hence the dosing control unit 28 may be configured to adjust the position of the valve member 310 so as to increase the amount of sustainable aviation fuel being incorporated into the fuel mixture.

In other scenarios in which the user wishes to prioritise aircraft performance during a given flight phase, the dosing control unit 28 may instead be configured to prioritise the supply of the high-performance aircraft fuel to the fuel consuming device 24 and hence the dosing control unit 28 may be configured to adjust the position of the valve member 310 so as to increase the amount of high-performance aircraft fuel being incorporated into the fuel mixture.

In this manner, the composition of the fuel mixture being supplied to the fuel consuming device 24 can be actively adjusted (or tailored) via the dosing control unit 28 to help ensure that the supplied fuel mixture is optimised to meet the priorities of the operator during a given flight phase.

In other embodiments, the second tank sensor 38 may be a viscosity sensor or a microbial sensor (as has been described above with reference to the first tank sensor 36) or may be a different type of sensor configured to monitor another parameter associated with the aircraft fuel stored in the second tank 16.

It shall also be appreciated that in some embodiments, the second tank 16 may comprise a plurality of sensors configured to monitor a plurality of parameters associated with the aircraft fuel (or the fuel additive) stored therein.

The aircraft fuel dosing system 10 may further comprise a third tank sensor 40 located in the third tank 32 for monitoring one or more parameters associated with fuel additive contained therein.

In the illustrated embodiment, the third tank sensor 40 is a composition sensor configured to monitor a chemical composition of the fuel additive stored in the third tank 32 and subsequently output a signal which is indicative of said composition to the dosing control unit 28.

It shall be appreciated that the third tank sensor 40 may communicate with the dosing control unit 28 via wired or wireless communication means.

In some embodiments, the dosing control unit 28 may comprise one or more processors configured to determine (or verify) the composition of the fuel additive stored in the third tank 32 based on the output signals received from the third tank sensor 40.

The dosing control unit 28 may be further configured to adjust the amount of the fuel additive incorporated into the fuel mixture based on the chemical composition of the fuel additive contained in the third tank 32 to account for variations in fuel additive composition so as to ensure that a desired fuel ratio is being delivered to the fuel consuming device 24.

The dosing control unit 28 may also be configured to activate the shut-off valve 300 so as to shut-off the supply of the fuel mixture to the fuel consuming device 24 in the event that the one or more processors determine that an incorrect fuel additive has been added to the third tank 32.

In other embodiments, the third tank sensor 40 may be a viscosity sensor or a microbial sensor (as has been described above with reference to the first tank sensor 36) or may be a different type of sensor configured to monitor another parameter associated with the fuel additive stored in the third tank 32.

It shall also be appreciated that in some embodiments, the third tank 32 may comprise a plurality of sensors configured to monitor a plurality of parameters associated with the fuel additive stored therein.

Figure 8 shows a schematic representation of an aircraft fuel dosing system 10 according to another embodiment of the present invention.

It shall be appreciated that the aircraft fuel dosing system 10 depicted in Figure 8 has many features in common with the aircraft fuel dosing system 10 depicted in Figure 7B and so, for the sake of conciseness, only the differences shall be described herein. Like components have been denoted by corresponding reference numerals.

The aircraft fuel dosing system 10 illustrated in Figure 8 further comprises a fuel blending device sensor 42 located in the fuel blending device 20 for monitoring one or more parameters associated with the fuel mixture contained in the fuel blending device 20.

In the illustrated embodiment, fuel blending device sensor 42 is a composition sensor configured to monitor a chemical composition of fuel mixture contained in the fuel blending device 20 and subsequently output a signal which is indicative of said composition to the dosing control unit 28.

It shall be appreciated that the fuel blending device sensor 42 may communicate with the dosing control unit 28 via wired or wireless communication means.

The dosing control unit 28 may comprise one or more processors configured to determine the composition of the fuel mixture contained in the fuel blending device 20 based on the output signal received from the fuel blending device sensor 42.

The dosing control unit 28 may be further configured to adjust a composition of the fuel mixture based on the output signal received from the fuel blending device sensor 42 to help ensure that a desired fuel ratio is achieved within the fuel blending device 20.

For example, the dosing control unit 28 may be configured adjust the amount of aircraft fuel (or fuel additive) incorporated into the fuel mixture based on the chemical composition of the fuel mixture contained in the fuel blending device 20 to account for variations in fuel composition and to thereby ensure that a desired fuel ratio is being delivered to the fuel consuming device 24.

The dosing control unit 28 may also be configured to activate the shut-off valve 300 so as to shut-off the supply of the fuel mixture to the fuel consuming device 24 in the event that the one or more processors determine that the chemical composition of the fuel mixture contained within the fuel blending device 20 is incorrect.

The aircraft fuel dosing system 10 may also continue to periodically or continuously monitor and/or adjust the composition of the fuel mixture contained within the fuel blending device 20 to ensure that the desired fuel ratio is maintained throughout the operation of the aircraft.

In other embodiments, the fuel blending device sensor 42 may be a viscosity sensor or a microbial sensor (as has been described above with reference to the first tank sensor 36) or may be a different type of sensor configured to monitor another parameter associated with the fuel blending device 20.

For example, in some embodiments, the fuel blending device sensor 42 may be an electrical sensor configured to monitor the build-up of electric charge (or static) within the fuel blending device 20 and subsequently output a signal which is indicative of said static levels to the dosing control unit 28.

The dosing control unit 28 may comprise one or more processors which may be configured to compare the output signal received from the fuel blending device sensor 42 with a pre-determined aircraft static threshold.

In the event that the output signal received from the fuel blending device sensor 42 falls outside the aforementioned aircraft static threshold, the one or more processors may be further configured to generate an error signal and determine a necessary control action in order to minimize the error signal.

For example, in some embodiments, the second 16 and/or third 32 tanks may contain a static dissipator. As such, upon determining that the static level of the fuel blending device 20 falls outside a pre-determined aircraft static threshold, the dosing control unit 28 may send a control action (or control signal) to the valve actuator 320 so as to increase the amount of the static dissipator being supplied to the fuel mixture to help ensure that the static level of the fuel blending device 20 returns back within the pre-determined aircraft static threshold.

Once the static level has been brought back within the pre-determined aircraft static threshold, the process of monitoring, comparing, generating error, and adjusting the control action may be repeated periodically or continuously to ensure that an acceptable static level is maintained throughout the operation of the aircraft.

It shall also be appreciated that in some embodiments, the fuel blending device 20 may comprise a plurality of sensors configured to monitor a plurality of parameters associated with the fuel blending device 20 and/or the fuel mixture contained therein.

The aircraft fuel dosing system 10 may further comprise a delivery line sensor 44 located in the delivery line 22 for monitoring one or more parameters associated with the fuel mixture being delivered to the fuel consuming device 24.

In the illustrated embodiment, delivery line sensor 44 is a composition sensor configured to monitor a chemical composition of fuel mixture in the delivery line 22 and subsequently output a signal which is indicative of said composition to the dosing control unit 28.

It shall be appreciated that the delivery line sensor 44 may communicate with the dosing control unit 28 via wired or wireless communication means.

In some embodiments, the dosing control unit 28 may comprise one or more processors configured to determine the composition of the fuel mixture contained in the delivery line 22 based on the output signal received from the delivery line sensor 44.

The dosing control unit 28 may be further configured to adjust a composition of the fuel mixture based on the output signals received from delivery line sensor 44 to help ensure that the fuel mixture being delivered to the fuel consuming device 24 has the desired fuel ratio.

For example, the dosing control unit 28 may be configured adjust the amount of the aircraft fuel (or the fuel additive) incorporated into the fuel mixture based on the chemical composition of the fuel mixture contained in the delivery line 22 to account for variations in fuel composition so as to ensure that a desired fuel ratio is being delivered to the fuel consuming device 24.

The dosing control unit 28 may also be configured to activate the shut-off valve 300 so as to shut-off the supply of the fuel mixture to the fuel consuming device 24 in the event that the one or more processors determine that the chemical composition of the fuel mixture contained within the delivery line 22 is incorrect.

The aircraft fuel dosing system 10 may continue to periodically or continuously monitor the composition of the fuel mixture in the delivery line 22 to ensure that the desired fuel ratio is being delivered to the fuel consuming device 24 throughout the operation of the aircraft.

In other embodiments, the delivery line sensor 44 may be a viscosity sensor or a microbial sensor (as has been described above with reference to the first tank sensor 36) or may be a different type of sensor configured to monitor another parameter associated with the fuel mixture contained in the delivery line 22.

It shall also be appreciated that in some embodiments, the delivery line 22 may comprise a plurality of sensors configured to monitor a plurality of parameters associated with the fuel mixture being delivered to the fuel consuming device 24.

Where the word 'or' appears this is to be construed to mean 'and/or' such that items referred to are not necessarily mutually exclusive and may be used in any appropriate combination.

Although the invention has been described above with reference to one or more preferred embodiments, it will be appreciated that various changes or modifications may be made without departing from the scope of the invention as defined in the appended claims.

## Claims

1. An aircraft fuel dosing system comprising:
a fuel blending device;
a first tank for storing an aircraft fuel;
a second tank for storing a further aircraft fuel or a fuel additive;
a first line configured to transport the aircraft fuel from the first tank to the fuel blending device;
a second line configured to transport the further aircraft fuel or the fuel additive from the second tank to the fuel blending device such that the further aircraft fuel or the fuel additive from the second tank can be combined with the aircraft fuel from the first tank to create a fuel mixture;
a delivery line configured to transport the fuel mixture from fuel blending device to an aircraft fuel consuming device;
at least one sensor configured to monitor at least one parameter associated with the aircraft; and
a dosing control unit configured to receive an output signal from the at least one sensor indicative of the at least one parameter, and being further configured to automatically adjust a composition of the fuel mixture based on the received signal.

2. The aircraft fuel dosing system according to claim 1, wherein the at least one sensor comprises an emission sensor configured to monitor at least one parameter associated with an exhaust gas exhausted from the aircraft fuel consuming device.

3. The aircraft fuel dosing system according to claim 2, wherein the at least one parameter is selected from one or more of: a carbon (soot) content of the exhaust gas; a sulphur content of the exhaust gas; an oxygen (O₂) content of the exhaust gas; a nitrous oxide (NOₓ) content of the exhaust gas, and/or a non-volatile particulate matter (nvPM) content of the exhaust gas.

4. The aircraft fuel dosing system according to any preceding claim, wherein the at least one sensor comprises a first tank sensor configured to monitor at least one parameter associated with the aircraft fuel stored in the first tank;
preferably wherein the first tank sensor is a composition sensor configured to monitor a chemical composition of the aircraft fuel stored in the first tank; or
preferably wherein the first tank sensor is a microbial sensor configured to detect the presence of one or more microorganisms (e.g., bacteria) within the aircraft fuel stored in the first tank; or
preferably wherein the first tank sensor is a viscosity sensor configured to monitor a viscosity of the aircraft fuel contained within the first tank.

5. The aircraft fuel dosing system according to any preceding claim, wherein the at least one sensor comprises a second tank sensor configured to monitor at least one parameter associated with the aircraft fuel, or the fuel additive, stored in the second tank;
preferably wherein the second tank sensor is a composition sensor configured to monitor a chemical composition of the further aircraft fuel, or the fuel additive, contained within the second tank.

6. The aircraft fuel dosing system according to any preceding claim, wherein the at least one sensor comprises a fuel blending device sensor configured to monitor at least one parameter associated with the fuel mixture contained in the fuel blending device;
preferably wherein the fuel blending device sensor is a composition sensor configured to monitor a chemical composition of the fuel mixture contained in the fuel blending device.
preferably wherein the fuel blending device sensor is an electrical sensor configured to monitor the build-up of electrical charge within the fuel blending device.

7. The aircraft fuel dosing system according to any preceding claim, wherein the at least one sensor comprises a delivery line sensor configured to monitor at least one parameter associated with the fuel mixture contained in the delivery line;
preferably wherein the delivery line sensor is a composition sensor configured to monitor a chemical composition of the fuel mixture contained in the delivery line.

8. The aircraft fuel dosing system according to any preceding claim, wherein the at least one sensor comprises a power rating of less than 100 milliampere (mA).

9. The aircraft fuel dosing system according to any preceding claim, further comprising a shut-off valve for controlling delivery of the fuel mixture to the aircraft fuel consuming device;
preferably wherein the fuel blending device is located upstream of said shut-off valve; or
preferably wherein the shut-off valve comprises:
a valve housing having an internal valve chamber;
a first inlet in fluid communication with the first line for introducing the aircraft fuel into the internal valve chamber;
a second inlet in fluid communication with the second line for introducing the further aircraft fuel or fuel additive into the internal valve chamber; and
an outlet port in fluid communication with the delivery line; and
wherein said internal valve chamber constitutes the fuel blending device of the aircraft fuel dosing system.

10. An aircraft comprising the aircraft fuel dosing system according to any preceding claim.

11. A method of automatically adjusting a composition of a fuel mixture on-board an aircraft, said method comprising the steps of:
providing a first tank containing an aircraft fuel;
providing a second tank containing a further aircraft fuel or a fuel additive;
mixing the aircraft fuel or the fuel additive from the second tank with the aircraft fuel from the first tank within a fuel blending device to create a fuel mixture;
monitoring at least one parameter associated with the aircraft;
automatically adjusting a composition of the fuel mixture based on the at least one parameter; and
delivering the fuel mixture to an aircraft fuel consuming device.

12. The method according to claim 11, wherein the at least one parameter is a parameter associated with the aircraft fuel and/or the fuel additive stored within the first and/or second tanks.

13. The method according to any of claims 11 or 12, wherein the at least one parameter is a parameter associated with the fuel blending device.

14. The method according to any of claims 11 to 13, wherein the at least one parameter is a parameter associated with the fuel mixture;
preferably wherein the at least one parameter is a chemical composition of the fuel mixture.

15. The method according to any of claims 11 to 14, wherein the at least one parameter is a parameter associated with an exhaust gas exhausted from the aircraft fuel consuming device, and optionally wherein the at least one parameter is selected from one or more of: a carbon (soot) content of the exhaust gas; a sulphur content of the exhaust gas; an oxygen (O₂) content of the exhaust gas; a nitrous oxide (NOₓ) content of the exhaust gas, and/or a non-volatile particulate matter (nvPM) content of the exhaust gas.
